# EUROPEAN PATENT SPECIFICATION

(11) **EP 1 054 691 B1**
(45) Date of publication and mention of the grant of the patent: **16.04.2003**
(21) Application number: 99906732.5
(22) Date of filing: 03.02.1999
(51) Int. Cl.: A61K 39/395, A61K 7/48, A61F 13/15, A61L 15/32

(54) **Anti-enzyme antibodies for the treatment of the skin**
Anti-Enzym Antikörper enthaltende Zusammensetzungen für die Behandlung der Haut
Compositions pour le traitment de la peau comprenant des anticorps anti-enzymes

(30) Priority: 18.02.1998 EP 98200507
(43) Date of publication of application: 29.11.2000
(73) Proprietor: The Procter & Gamble Company, Cincinnati, Ohio 45202 (US)
(72) Inventor: HERBOTS, Ivan, Maurice, Alfons, Jan, B-9230 Wetteren (BE); WEVERS, Jean, B-1840 Steenhuffel (BE); VAN HAUWERMEIREN, Tim, Joris, B-1880 Ramsdonk (BE)
(74) Representative: Hirsch, Uwe Thomas
(86) International application number: US9902396
(87) International publication number: WO99042131

(56) References cited:
- EP-A- 0 117 613
- EP-A- 0 562 864
- WO-A-97/17446
- US-A- 5 681 722
- PATENT ABSTRACTS OF JAPAN vol. 095, no. 007, 31 August 1995 & JP 07 109300 A (TEIJIN LTD), 25 April 1995

## Description

The invention relates to compositions for treatment of enzymatic dermatitis.

### Background

Various types of enzymes are present in the our body or on our skin, including enzymes from bacteria's, enzymes derived from our diet and enzymes produced by the body.

It is know that some of these enzymes can have undesirable side-effects. For example, lipase enzymes are present in the body exudates and these enzymes are known to cause dermatitis or skin rash, which for example, has always been a problem encountered by the users of disposable absorbent articles, such as diapers, incontinence articles, sanitary towels, training pants etc. When the skin is exposed to, for example, lipase enzymes in the body exudate, the lipid-containing components of the skin can be affected by these enzymes. The protection or barrier function of the top layer of the skin (the Strateum Corneum) will thus be diminished. This can effect the health of the skin and/or facilitate the infection of the skin. This can thus lead to skin or diaper rash.

Manufacturers of diapers and skin care products have developed various products over the past decades which help reduce the occurrence of diaper rash (or skin rash). The main focus thereby has been to reduce the exposure of the skin to the body exudates. This is for example done by introduction to the diaper of absorbing or better absorbing materials. The amount of water which is in contact with the skin is thus reduced.

EP 0117632-B relates to disposable articles which comprise lipase inhibiting agents, preferably zinc containing components, and a vehicle material. US 3,091,241 relates to the use of triacetine in vaginal tampons to inhibit lipase enzyme activity. US 3, 961,486 teaches the use of adipic acid to reduce the lipase enzyme activity and to reduce the skin rash.

The inventors now have found that the enzyme activity of enzymes, such as lipase in contact with the skin can effectively be reduced by specific antibodies. Thus, when the antibodies are applied to the skin, the enzymes present can be effectively inhibited or inactivated, whereby the development of enzymatic dermatitis can be reduced or avoided.

### Summary of the Invention

The invention provides the use of an antibody for preparation of a composition for treatment of enzymatic dermatitis of the external skin.

The invention also relates to absorbent articles comprising a composition containing an antibody.

### Detailed Description of the Invention

The antibodies herein are used for preparation of a composition for treatment of enzymatic dermatitis of the external skin, caused by the enzyme activity of enzymes present in the exudates, such as esterase enzymes, including lipase enzymes.

Lipase, lipase enzyme or lipolytic enzyme is the trivial or common term employed to represent a group of enzymes belonging to the esterases.

Their general activity is to hydrolyse fats present in the ester form (such as the glycerides found in human skin), and accordingly generate fatty acids and glycerol. Because this group of enzymes is so widely distributed in plants, moulds, bacteria, milk, and milk-products, as well as in almost all animal tissues, and because moreover human lipase enzymes are present in the pancreatic exudates, they are almost always present in body exudates,

The activity of lipase enzymes contributes to almost all skin rash, or in particular diaper rash, causing irritation by the digestive degenerative action of these enzymes on the skin per se and by breaking down the lipid-skin components, compromises the barrier property of the skin in the affected area. This breakdown of the integrity of the skin allows other components of the body exudates (urine and faeces in particular), which may not, by themselves, be irritating, to migrate through the compromised skin. At this point normally harmless components may then become irritating.

By "treatment" is meant herein an improvement of the affected condition of the human or animal body, caused by the enzyme activity. This thus includes the reduction or at least stabilisation of the enzymatic dermatitis or the rash of the skin, caused by enzymes.

The amount of the composition of the invention used for the reduction of the enzyme activity or in the treatment, will vary with the particular location of the condition being treated, the severity of the condition being treated, the expected duration of the treatment, any specific sensitivity to either the composition specific to the user, the condition of the user, concurrent therapies being administered, other conditions present in the user.

For the present invention it is preferred that a minimum inhibitory concentration of the compositions containing the antibodies is, preferably topically, applied in an amount and form such that it is available to inhibit the activity of the enzymes present.

### Antibodies

An essential element of the present invention is an enzyme-directed antibody, herein referred to as antibody.

The immunoglobulins are classified into 5 classes, respectively : IgM, IgG, IgA, IgD and IgE. Preferred types of immunoglobulins are IgG and IgA. Secretory slgA which are found into human excreted body fluids such as milk, saliva, respiratory and intestinal fluids are especially designed to survive in said secretions, they have enhanced binding characteristics and are resistant to proteolytic hydrolysis.

The antibody which may be monoclonal or polyclonal or an antibody fragment, may be generated by techniques conventional in the art, for example by using recombinant DNA techniques allowing to produce antibodies variants with new properties : reduced immunogenecity, enhanced affinity, altered size. Specific binding may also be used. Preferred for the purpose of the present invention is a monoclonal antibody, more preferred is a fragment thereof. These fragments may be similarly generated by conventional techniques such as enzymatic digestion by papain or pepsin, or using recombinant DNA techniques. Antibody fragments may also be generated by conventional recombinant DNA techniques. Antibodies and antibodies' fragments may be humanised, such as described in Meded. - Fac. Landbouwkd. Toegepast Biol. Wet. (Univ. Gent) (1995), 60(4a, Forum for Applied Biotechnology, 1995, Part 1), 2057-63.

Heavy and light chains are indeed composed of constant and variable domains. In the organisms producing immunoglobulins in their natural state the constant domains are very important for a number of functions, but for many applications in industrial processes and products their variable domains are sufficient. Consequently many methods have been described to produce antibody fragments.

Antibody fragments which can be preferred herein. Typically, they may be a Fab, a Fv, a scFv or any other fragment having similar binding properties. Preferred routes to antibodies fragments are through recombinant DNA technology, so that the fragment is expressed by a genetically transformed organism.

Antibodies and antibody fragments produced by recombinant DNA technology do not need to be identical to fragment of antibodies produced in vertebrates, having nevertheless the same binding properties evaluated by their Km, Ki and Kcat. For instance they may include sequences of amino acids and/or glycosylations which differ from those found in antibodies produced in other ways, especially sequences at the end of fragments. Somewhat analogously, antibody fragments produced through recombinant DNA technology may include extra amino acid sequences at their termini which have no counterpart in antibodies produced in other ways.

A related possibility is that a binding agent for use in this invention is a natural or synthetic polymer which mimics the specific binding activity of a natural antibody's complementary region(s). Such a polymer is for example a polypeptide or a polymer imprinting (Angew. Chem. Int. Ed. Engl. 1995, 34, 1812-1832).

The usual method for the production of antibodies may be adopted in immunising mammals or poultry with the corresponding antigens. As mammals to be immunised, mouse, rabbits, goats, sheep, horses, cows, etc. may be used. The antibody (immunoglobulin fraction) may be separated from the antiserum, the milk or the eggs according to the ordinary antibody purification method including salting-out method, Poison extraction, gel-filtration chromatography, ion-exchange chromatography, affinity chromatography and the like, the salting-out method using ammonium sulfate to produce the precipitates, followed by dialysing the precipitates against physiological saline to obtain the purified precipitates as the antibody.

Plants are also capable of synthesising and assembling every kind of antibody molecule and allow a large scale of production of antibodies as described in Tibtech. Dec 1995, Vol 13, pp 522-527; Plant Mol. Biol., 26, pp 1701-1710, *1994* and Biotechnol. proj. *1991*, 7, pp 455-461 and in US patent 5, 202,422. Antibodies can also be produced into microorganisms such as E. coli or S. cerevisiae via biofermentation process as illustrated in the EP patent 667 394.

Techniques for the production of antibody fragments are well known in the literature: Saiki et al. Science 230 1350-54 (1985); Orlandi et al. PNAS USA 86 3833-7 (1989); WO89/09825; EP 368 684; WO 91/08482 and WO94/25591.

The drawbacks due to prolonged activity of the enzyme can be avoided by an effective control of the enzymatic activity trough the introduction of the specifically corresponding antibody. Such antibodies can be either polyclonal - directed to the whole enzyme structure - or monoclonal - directed to specific epitopes of the enzyme activity controlling regions of the enzyme structure. Antibodies raised against specific enzyme can effectively deactivate the enzyme by the antibody-antigen binding in or very near the active site. The formation of such complex leads to the enzyme deactivation and could be explained by the distortion of the 3-dimensional structure and/or steric hindrance at the substrate cleft.

Typically, the antibody is present in the composition in an amount such that in use, the molecular ratio of antibody to enzyme will be of 100:1 or lower, preferably of 50:1 or lower. For monoclonal antibodies or fragment thereof, the molecular ratio of antibody to enzyme will be generally of 50:1 or lower, preferably of 20:1 or lower.

### Method of preparation of the compositions

The compositions can be prepared by any method known in the art for preparation for cosmetic compositions or medicament. The exact method will depend on the nature of the composition. The antibodies can be added to the composition, combined with other ingredients commonly used in cosmetic compositions or medicaments, or dispersed or dissolved in water or oil or a water-in-oil emulsion prior to addition to the composition.

### Method of use of compositions

The composition of the invention can be applied to the skin which will be in contact with, or the vicinity of the enzymes.

The compositions is preferably applied (firstly) to an absorbent article, such as a diaper wipe or tissue, which will then be applied to the skin.

### Absorbent Articles

The compositions of the present invention can be comprised in an absorbent article, preferably a disposable absorbent article. Particularly preferred absorbent articles therefor is a wipe or a diaper.

As used herein, the term "absorbent articles" refers to devices which absorb and contain body exudates, and, more specifically, refers to devices which are placed against or in proximity to the body of the wearer to absorb and contain the various exudates discharged from the body. The term "disposable" is used herein to describe absorbent articles which are not intended to be laundered or otherwise restored or reused as an absorbent article (i.e., they are intended to be discarded after a single use and, preferably, to be recycled, composted or otherwise disposed of in an environmentally compatible manner).

The structure of the disposable absorbent article is not critical to the practice of the present invention.

Normally, the composition is incorporated into the absorbent article or diaper in particular in an amount which will deliver the required treatment or reduction or inhibition of the enzyme activity, whereby it may be preferred that this is achieved after frequent use.

The disposable absorbent article preferably contains the composition according to the invention at a level such that the antibodies therein are present (based on active antibody materiel) at a level of from 0.00001% to 5%, more preferably from 0.0001% to 1%, most preferably from 0.001% to 0.5% by weight of the article.

### Additional ingredients

The composition of the invention can optionally comprise additional ingredients. Which ingredients are present and at which level depends on the character of the composition and the use thereof.

It can be preferred that the compositions comprise one or more other ingredient which can reduce dermatitis, or compounds which can help the healing of the skin, such as vitamins (vitamin E) and cortisone, and also compounds to soften the skin such as vaseline, glycerin, triethyleneglycol, lanolin, paraffin and another group of polymers extensively employed by pharmaceutical and cosmetic manufactures, as also described herein.

A preferred compound, which can aid the reduction of the lipase activity and which is suitable for use in the preparation of the compositions of the present invention or for use in the compositions, can be an ester compound of the formulation: 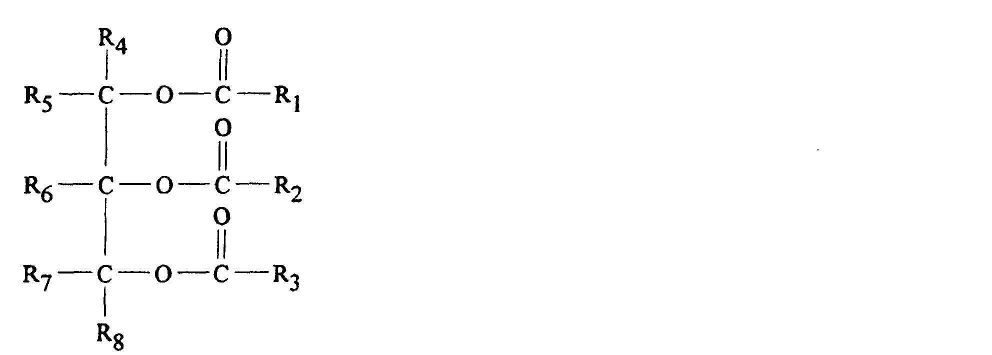 wherein R₁, R₂ and R₃ are independently an alkyl or alkenyl or hydroxyalkyl group with from 1 to 22 carbon atoms, and R₄, R₅, R₆, R₇ and R₈ are independently selected from the group consisting of C₁-C₁₀ linear or branched alkyl, alkenyl or hydroxyalkyl groups, hydroxy, chloride, bromide, amine or hydrogen.

Highly preferred are the compounds above wherein R₄, R₅, R₆, R₇ and R₈ of said compound are hydrogen and preferably wherein R₁, R₂ and R₃ are independently an C1-C4 alkyl group.

Such a preferred compound can be glycerol triacetate.

It has been found that another highly preferred additional component to be used for the preparation of the compositions of the present invention or for use in the compositions of the present invention are certain cationic compounds.

Another preferred ester compound for use in, or for preparation of the compositions of the invention is of the formulation: 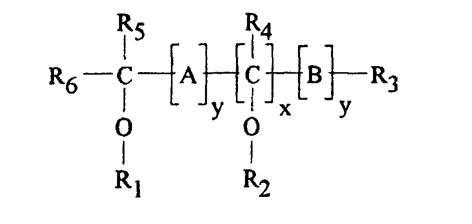 or 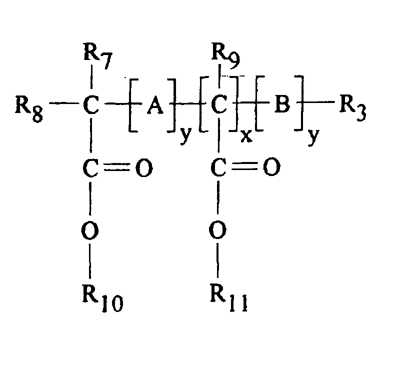 wherein R₁ and each R₂ independently are an acyl group with from 2 to 22 carbon atoms, or an alkyl, alkenyl, arylalkyl, hydroxyalkyl group with from 1 to 24 carbon atoms or hydrogen, whereby at least one of R₁ and R₂ is such an acyl group, R₃ R₄, R₅, R₆, R₇, R₈, and R₉ are independently an alkyl, alkenyl, arylalkyl, hydroxyalkyl, alkoxy groups of from 1 to 24 carbon atoms, hydroxy group or hydrogen; R₁₀ and R₁₁ are independently an alkyl, alkenyl, arylalkyl, hydroxyalkyl, alkoxy groups of from 2 to 24 carbon atoms, hydroxy group or hydrogen; A and B are independently a C₁-C₆ linear or branched alkylene, alkenylene, alkoxylene, hydroxyalkylene groups; the values of x are independently from 0 to 15; the values of y are independently 0 or 1, with the proviso that when x =2 and y=0, at least one R₂ is an alkyl, alkenyl, arylalkyl, hydroxyalkyl group with from 1 to 24 carbon atoms or hydrogen

Preferred are the ester compounds as defined above wherein x is 1 or 2, y is 0; R₁ and one R₂ are a C₂-C₁₆ acyl group, R₁₀ and one or more R₁₁ are a C₂-C₁₆ alkyl group; R₃, R₄, R₅, R₆, R₇ and R₈ are hydrogen

It is highly preferred that the additional ester compound is a mono or diester of formula (V), most preferably a mono or diester of citric acid or tartaric acid (or salts thereof), or a triester of citric acid.

Also certain cationic compounds can be useful as additional ingredients of the compositions of the invention are preferably of the of formulations: 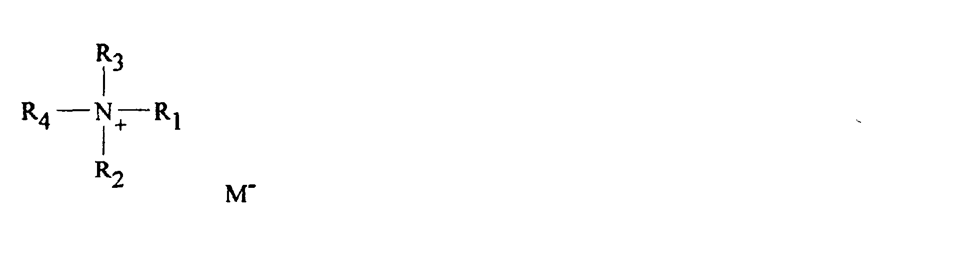 or 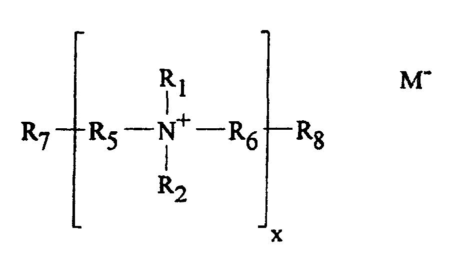 or an amphoteric compound and preferably an acidity source, the amphoteric compound having at its iso-electric point the formula: 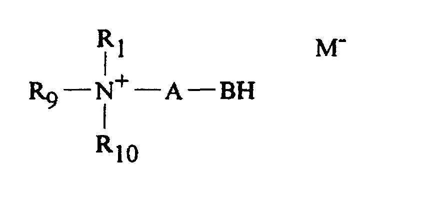 wherein R₁, R₂, R₃ and R₄ are independently a C₁-C₂₂ alkyl, alkenyl, aryl, arylalkyl, amidoalkyl, (poly) alkoxy, hydroxyalkyl, or acyl groups, or two or more groups of R₁, R₂, R₃ and R₄ form together one or more ring structures; R₅, R₆ and A are independently a C₁-C₂₂ alkylene, alkenylene, (poly) alkoxylene, hydroxyalkylene, arylalkylene or amido alkylene groups; R₇ and R₈ are independently a C₁-C₄ alkyl, alkenyl, alkoxy group or a hydroxy group or hydrogen; R₉ and R₁₀ are independently a C₁-C₂₂ linear or branched alkyl, alkenyl, aryl, arylalkyl, amidoalkyl, (poly) alkoxy, hydroxyalkyl, or acyl groups, or two or more of the groups R₁, R₉ and R₁₀ form together one or more ring structures; BH is a proton donating group; x is from 2 to 4; and M- is a counter ion.

It should be understood that for the purpose of this invention, the groups R₁-R₁₀ can be substituted by any appropriate substituent group.

R₁, R₂, R₃ and R₉ are independently preferably C₁-C₈, more preferably C₁-C₄ alkenyl or alkoxy, more preferably alkyl groups, most preferably methyl or ethyl groups.

Preferably, R₄, R₅ and R₁₀ are independently C₈-C₁₈, more preferably C₁₂-C₁₆ alkenyl or alkoxy, more preferably alkyl or arylalkyl groups, whereby it can be preferred that one of the R₄, R₅ and R₉ substituents is benzyl group.

Alternatively, it can be preferred that the cationic compound comprises atleast one R₁, R₂ or R₃ or R₉ being a poly alkoxy group.

Thus, R₁, R₂, R₃ and R₉ preferably are independently polyalkoxy groups comprising C₂-C₆, preferably C₂-C₃ alkoxy units and having an alkoxylation number of from 2 to 50, preferably from 5 to 18. Then, R₃, R₄ and R₁₀ are independently preferably C₁-C₈, more preferably C₁-C₄ alkenyl or alkoxy, more preferably alkyl groups, most preferably methyl or ethyl groups.

A, R₅ and R₆ are, independently, preferably C₁-C₆ alkenylene or more preferably alkylene groups, most preferably methylene or ethylene.

Preferred compounds can be benzalkonium chloride or Merquat 2200 (Trade name, being a 2-Propeneamide polymer of N,N-dimethyl-N-2-Propenyl-1-amonium chloride).

Preferred cationic compounds of the formulas above comprise one or more substituted R₁, R₂, R_{3,}R₄, R₉ or R₁₀ groups and/ or a substituted R₅ and/or substituted R₆ group, whereby the substituent is selected from the group from the group consisting of derivatives of silicon, glucose, fructose and saccharose.

Preferred can be Glucquat 125 (trade name, being lauryl dimethyl glucet-10-hydroxydimonium chloride).

The additional cationic compounds or additional triester compounds are preferably present in the compositions of the invention at a level of from 0.01% to 20%, more preferably from 0.05% to 10%, most preferably from 0.1% to 5% by weight of the composition.

### Lotions, creams, oils, foams, ointments, gels, powders, tablets and the like

The present compositions can be used for any suitable purpose. In particular, the present compositions are suitable for topical application to the skin or hair. In particular, the skin care compositions can be in the form of creams, sprays, lotions, gels, and the like. Preferably the cosmetic compositions herein are in the form of an oil-in-water emulsion of one or more oil phases in an aqueous continuous phase, each oil phase comprising a single oily component or a mixture of oily components in miscible or homogeneous form but said different oil phases containing different materials or combinations of materials from each other. The overall level of oil phase components in the compositions of the invention is preferably from about 0.1% to about 60%, preferably from about 1% to about 30% and more preferably from about 1% to about 10% by weight.

The compositions of the invention are preferably in the form of a moisturising cream or lotion, which can be applied to the skin as a leave-on product.

### Method to assess antibodies activity

The lipase activity was determined by using a kit from SIGMA (Cat. No. 805A). The test was carried out with diluited faecal matter (1/10 w/v). The glycerol released after hydrolysation of monoglycerides is transformed to glycerol-3-phosphate which is subsequently oxidized to dihydroxyacetone phosphate and hydrogen peroxide. The peroxide formed reacts with 4-aminoantipirine and sodium-N-ethyl-N-(2-hydroxy-3-sulfopropyl)-m-toluidine in th epresence of peroxidase to form a quinone diimine dye. The dye absorbs light at 550 nm. The rate of increase in absorbance is directly proportional to the lipolitic activity in the sample.

Tests are performed both in absence (control) and in presence of the specific antibodies. 0.5 ml of antibodies solution (5 % in water) are added into 10 ml of control and further incubated at 37 C. After 5 min and 120 min samples are taken to determine the enzyme activity.

| **Lipase (activity expressed as enzyme units/g)** | | | | |
|---|---|---|---|---|
| | 5 min | | 120 min | |
| | absorbance | activity | absorbance | activity |
| Control | 0.443 | 62.2 | 0.445 | 0.64 |
| Control + antibodies | 0.045 | 0.24 | 0.05 | 0.25 |

## Claims

1. The use of an enzyme-directed antibody for preparation of a composition for treatment of enzymatic dermatitis of the external skin.

2. The use as in claim 1, whereby the antibody is and esterase-enzyme directed antibody and the dermatitis is caused by the enzyme activity of esterase enzymes.

3. Use according to claim 1 or 2 wherein the composition is for prevention or treatment of diaper rash.

4. A use according to claim 1 or 2 or 3 wherein said antibody is a monoclonal antibody, preferably a fragment thereof.

5. A disposable absorbent article containing a composition containing an esterase enzyme-directed antibody.

6. A disposable absorbent article, according to claim 5, whereby the antibody is present at a level of from 0.00001 to 5% by weight of the articles, preferably 0.00001 to 1% by weight of the article.

7. A disposable absorbent article according to claims 5 or 6 in the form of a diaper.

8. A disposable diaper according to claims 5 to 7 comprising a topsheet, which contains the composition.

9. Use according to any of claims 1 to 4 in the form of a skin-care cream, lotion, gel, oil, ointment or powder, for topical application to the external skin or to a disposable absorbent article.

## Patentansprüche

1. Die Verwendung eines gegen ein Enzym gerichteten Antikörpers für das Zubereiten einer Zusammensetzung zur Behandlung enzymatischer Dermatitis der äußeren Haut.

2. Die Verwendung wie in Anspruch 1, wobei der Antikörper ein gegen ein Esterase-Enzym gerichteter Antikörper ist und die Dermatitis durch die Enzymaktivität von Esterase-Enzymen verursacht wird.

3. Verwendung gemäß Anspruch 1 oder 2, wobei die Zusammensetzung für die Prävention oder Behandlung von Windelausschlag ist.

4. Eine Verwendung gemäß Anspruch 1 oder 2 oder 3, wobei der Antikörper ein monoklonaler Antikörper, vorzugsweise ein Fragment davon, ist.

5. Ein saugfähiger Wegwerfartikel, enthaltend eine Zusammensetzung, die einen gegen ein Esterase-Enzym gerichteten Antikörper enthält.

6. Ein saugfähiger Wegwerfartikel gemäß Anspruch 5, wobei der Antikörper in einer Menge von 0,00001 bis 5 Gew.-% der Artikel, vorzugsweise 0,00001 bis 1 Gew.-% des Artikels, vorliegt.

7. Ein saugfähiger Wegwerfartikel gemäß Anspruch 5 oder 6 in Form einer Windel.

8. Eine Wegwerfwindel gemäß den Ansprüchen 5 bis 7, enthaltend eine obere Lage, welche die Zusammensetzung enthält.

9. Verwendung gemäß einem der Ansprüche 1 bis 4 in Form einer Hautpflegecreme, einer Lotion, eines Gels, eines Öls, einer Salbe oder eines Puders zur topischen Anwendung auf der äußeren Haut oder an einem saugfähigen Wegwerfartikel.

## Revendications

1. Utilisation d'un anticorps dirigé contre une enzyme pour la préparation d'une composition pour le traitement de la dermatite enzymatique de l'épiderme.

2. Utilisation selon la revendication I dans laquelle l'anticorps est un anticorps dirigé contre les enzymes estérases et la dermatite est provoquée par l'activité enzymatique dcs enzymes estérases.

3. Utilisation selon la revendication 1 ou 2 dans laquelle la composition sert à la prévention ou au traitement de l'érythème fessier.

4. Utilisation selon la revendication 1, 2 ou 3 dans laquelle ledit anticorps est un anticorps monoclonal, de préférence un fragment de ce dernier.

5. Article absorbant jetable contenant une composition contenant un anticorps dirigé contre les enzymes estérases.

6. Article absorbant jetable selon la revendication 5 dans lequel l'anticorps est présent à raison de 0,00001 à 5% cn poids de l'article, de préférence entre 0,00001 et 1% en poids de l'article.

7. Article absorbant jetable selon les revendications 5 ou 6 sous la forme d'une couche.

8. Couche jetable selon les revendications 5 à 7 comprenant une feuille de dessus contenant la composition.

9. Utilisation selon l'une quelconque des revendications 1 à 4 sous forme de crème, lotion, gel, huile, pommade ou poudre de traitement de la peau, pour application topique sur l'épiderme ou sur un article absorbant jetable.
